# DEMANDE DE BREVET EUROPEEN

(11) **EP 0 614 978 A1**
(43) Date de publication de la demande: **14.09.1994**
(21) Numéro de dépôt: 94400492.8
(22) Date de dépôt: 08.03.1994
(51) Int. Cl.: C12N 15/12, C07K 13/00, C12N 15/85, C12N 5/10, C12P 21/08, C12P 21/02, G01N 33/577, A61K 37/02

(54) **Nouveaux récepteurs Fc-gamma humains solubles, Leur procédé de préparation, les compositions pharmaceutiques les contenant, leur application comme médicaments et leur application diagnostique**

(30) Priorité: 09.03.1993 FR 9302675
(71) Demandeur: ROUSSEL UCLAF, F-93230 Romainville (FR); THE GENERAL HOSPITAL CORPORATION, Boston, MA 02114 (US)
(72) Inventeur: Aruffo, Alejandro, Edmonds, Washington 98020 (US); Seed, Brian, Boston 02114 Massachusetts (US); Fridman, Wolf H., F-75005 Paris (FR); Sautes, Catherine, F-75005 Paris (FR); Teillaud, Christophe, F-75013 Paris (FR); Teilland, Jean-Luc, F-75005 Paris (FR)
(74) Mandataire: Vieillefosse, Jean-Claude

(57) **Abrégé**

L'invention a pour objet un récepteur RFcγIII humain soluble ayant la séquence en acides aminés SEQ ID N°1 : 
Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60
Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val
éventuellement précédée d'une méthionine à l'extrémité N-terminale ainsi que les analogues de cette séquence.

LISTE DE SEQUENCES

(1) INFORMATION GENERALE:

(i) DEPOSANT:
(A) NOM: ROUSSEL UCLAF
(B) RUE: 35, Boulevard des Invalides
(C) VILLE: PARIS
(E) PAYS: FRANCE
(F) CODE POSTAL: 75007
(H) TELECOPIE: (33) 1.49.91.46.10

(A) NOM: The General Hospital Corporation
(B) RUE: Fruit Street
(C) VILLE: BOSTON
(D) PROVINCE: MASSACHUSETTS
(E) PAYS: UNITED STATES OF AMERICA
(F) CODE POSTAL: 02114

(ii) TITRE DE L' INVENTION: Nouveaux recepteurs Fc-gamma III humains
solubles, leur procede de preparation, les compositions
pharmaceutiques les contenant, leur application comme
medicaments et leur application diagnostique.

(iii) NOMBRE DE SEQUENCES: 6

(iv) FORME LISIBLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)
et WORDPERFECT 5.1 pour corrections informations 2 (ii)

(2) INFORMATION POUR LA SEQ ID NO: 1:

(i) CARACTERISTIQUES DE LA SEQUENCE:
(A) LONGUEUR: 194 acides aminés
(B) TYPE: acide aminé
(C) NOMBRE DE BRINS: simple
(D) CONFIGURATION: linéaire

(ii) TYPE DE MOLECULE: protéine

(vi) ORIGINE:
(A) ORGANISME: Homo sapiens

(xi) DESCRIPTION DE LA SEQUENCE: SEQ ID NO: 1:

Arg Thr Glu Asp Leu Pro Lys Ala Val Val Phe Leu Glu Pro Gln Trp
1               5                   10                  15

Tyr Ser Val Leu Glu Lys Asp Ser Val Thr Leu Lys Cys Gln Gly Ala
            20                  25                  30

Tyr Ser Pro Glu Asp Asn Ser Thr Gln Trp Phe His Asn Glu Ser Leu
        35                  40                  45

Ile Ser Ser Gln Ala Ser Ser Tyr Phe Ile Asp Ala Ala Thr Val Asn
    50                  55                  60

Asp Ser Gly Glu Tyr Arg Cys Gln Thr Asn Leu Ser Thr Leu Ser Asp
65                  70                  75                  80

Pro Val Gln Leu Glu Val His Ile Gly Trp Leu Leu Leu Gln Ala Pro
                85                  90                  95

Arg Trp Val Phe Lys Glu Glu Asp Pro Ile His Leu Arg Cys His Ser
            100                 105                 110

Trp Lys Asn Thr Ala Leu His Lys Val Thr Tyr Leu Gln Asn Gly Lys
        115                 120                 125

Asp Arg Lys Tyr Phe His His Asn Ser Asp Phe His Ile Pro Lys Ala
    130                 135                 140

Thr Leu Lys Asp Ser Gly Ser Tyr Phe Cys Arg Gly Leu Val Gly Ser
145                 150                 155                 160

Lys Asn Val Ser Ser Glu Thr Val Asn Ile Thr Ile Thr Gln Gly Leu
                165                 170                 175

Ala Val Ser Thr Ile Ser Ser Phe Ser Pro Pro Gly Ala Asp Pro Arg
            180                 185                 190

Leu Val

(2) INFORMATION POUR LA SEQ ID NO: 2:

(i) CARACTERISTIQUES DE LA SEQUENCE:
(A) LONGUEUR: 672 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: Homo sapiens

(ix) CARACTERISTIQUE ADDITIONELLE:
(A) NOM/CLE: CDS
(B) EMPLACEMENT: 34..669

(ix) CARACTERISTIQUE ADDITIONELLE:
(A) NOM/CLE: mat_peptide
(B) EMPLACEMENT: 88..669

(ix) CARACTERISTIQUE ADDITIONELLE:
(A) NOM/CLE: sig_peptide
(B) EMPLACEMENT: 34..87

(x) INFORMATION DE LA PUBLICATION:
(A) AUTEURS: Simmons, David
Seed, Brian
(B) TITRE: The Fc gamma receptor of natural killer cells
is a phospholipid-linked membrane protein
(C) REVUE: Nature
(D) VOLUME: 333
(F) PAGES: 568-570
(G) DATE: JUNE-9-1988
(K) RESIDUES PERTINENTS DANS LA SEQ ID NO: 2: DE 1 JUSQU'AU 650

(xi) DESCRIPTION DE LA SEQUENCE: SEQ ID NO: 2:

TCTTTGGTGA CTTGTCCACT CCAGTGTGGC ATC ATG TGG CAG CTG CTC CTC CCA 54
Met Trp Gln Leu Leu Leu Pro
-18 -15

ACT GCT CTG CTA CTT CTA GTT TCA GCT GGC ATG CGG ACT GAA GAT CTC 102
Thr Ala Leu Leu Leu Leu Val Ser Ala Gly Met Arg Thr Glu Asp Leu
-10 -5 1 5

CCA AAG GCT GTG GTG TTC CTG GAG CCT CAA TGG TAC AGC GTG CTT GAG 150
Pro Lys Ala Val Val Phe Leu Glu Pro Gln Trp Tyr Ser Val Leu Glu
10 15 20

AAG GAC AGT GTG ACT CTG AAG TGC CAG GGA GCC TAC TCC CCT GAG GAC 198
Lys Asp Ser Val Thr Leu Lys Cys Gln Gly Ala Tyr Ser Pro Glu Asp
25 30 35

AAT TCC ACA CAG TGG TTT CAC AAT GAG AGC CTC ATC TCA AGC CAG GCC 246
Asn Ser Thr Gln Trp Phe His Asn Glu Ser Leu Ile Ser Ser Gln Ala
40 45 50

TCG AGC TAC TTC ATT GAC GCT GCC ACA GTC AAC GAC AGT GGA GAG TAC 294
Ser Ser Tyr Phe Ile Asp Ala Ala Thr Val Asn Asp Ser Gly Glu Tyr
55 60 65

AGG TGC CAG ACA AAC CTC TCC ACC CTC AGT GAC CCG GTG CAG CTA GAA 342
Arg Cys Gln Thr Asn Leu Ser Thr Leu Ser Asp Pro Val Gln Leu Glu
70 75 80 85

GTC CAT ATC GGC TGG CTG TTG CTC CAG GCC CCT CGG TGG GTG TTC AAG 390
Val His Ile Gly Trp Leu Leu Leu Gln Ala Pro Arg Trp Val Phe Lys
90 95 100

GAG GAA GAC CCT ATT CAC CTG AGG TGT CAC AGC TGG AAG AAC ACT GCT 438
Glu Glu Asp Pro Ile His Leu Arg Cys His Ser Trp Lys Asn Thr Ala
105 110 115

CTG CAT AAG GTC ACA TAT TTA CAG AAT GGC AAA GAC AGG AAG TAT TTT 486
Leu His Lys Val Thr Tyr Leu Gln Asn Gly Lys Asp Arg Lys Tyr Phe
120 125 130

CAT CAT AAT TCT GAC TTC CAC ATT CCA AAA GCC ACA CTC AAA GAT AGC 534
His His Asn Ser Asp Phe His Ile Pro Lys Ala Thr Leu Lys Asp Ser
135 140 145

GGC TCC TAC TTC TGC AGG GGG CTT GTT GGG AGT AAA AAT GTG TCT TCA 582
Gly Ser Tyr Phe Cys Arg Gly Leu Val Gly Ser Lys Asn Val Ser Ser
150 155 160 165

GAG ACT GTG AAC ATC ACC ATC ACT CAA GGT TTG GCA GTG TCA ACC ATC 630
Glu Thr Val Asn Ile Thr Ile Thr Gln Gly Leu Ala Val Ser Thr Ile
170 175 180

TCA TCA TTC TCT CCA CCT GGC GCG GAT CCT AGA CTA GTC TAG 672
Ser Ser Phe Ser Pro Pro Gly Ala Asp Pro Arg Leu Val
185 190

(2) INFORMATION POUR LA SEQ ID NO: 3:

(i) CARACTERISTIQUES DE LA SEQUENCE:
(A) LONGUEUR: 212 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 3:

Met Trp Gln Leu Leu Leu Pro Thr Ala Leu Leu Leu Leu Val Ser Ala
-18 -15 -10 -5

Gly Met Arg Thr Glu Asp Leu Pro Lys Ala Val Val Phe Leu Glu Pro
1 5 10

Gln Trp Tyr Ser Val Leu Glu Lys Asp Ser Val Thr Leu Lys Cys Gln
15 20 25 30

Gly Ala Tyr Ser Pro Glu Asp Asn Ser Thr Gln Trp Phe His Asn Glu
        35                  40                  45

Ser Leu Ile Ser Ser Gln Ala Ser Ser Tyr Phe Ile Asp Ala Ala Thr
    50                  55                  60

Val Asn Asp Ser Gly Glu Tyr Arg Cys Gln Thr Asn Leu Ser Thr Leu
65 70 75

Ser Asp Pro Val Gln Leu Glu Val His Ile Gly Trp Leu Leu Leu Gln
80 85 90

Ala Pro Arg Trp Val Phe Lys Glu Glu Asp Pro Ile His Leu Arg Cys
95 100 105 110

His Ser Trp Lys Asn Thr Ala Leu His Lys Val Thr Tyr Leu Gln Asn
                115                 120                 125

Gly Lys Asp Arg Lys Tyr Phe His His Asn Ser Asp Phe His Ile Pro
            130                 135                 140

Lys Ala Thr Leu Lys Asp Ser Gly Ser Tyr Phe Cys Arg Gly Leu Val
        145                 150                 155

Gly Ser Lys Asn Val Ser Ser Glu Thr Val Asn Ile Thr Ile Thr Gln
    160                 165                 170

Gly Leu Ala Val Ser Thr Ile Ser Ser Phe Ser Pro Pro Gly Ala Asp
175                 180                 185                 190

Pro Arg Leu Val

(2) INFORMATION POUR LA SEQ ID NO: 4:

(i) CARACTERISTIQUES DE LA SEQUENCE:
(A) LONGUEUR: 21 paires de bases
(B) TYPE: acide nucléique
(C) NOMBRE DE BRINS: simple
(D) CONFIGURATION: linéaire

(ii) TYPE DE MOLECULE: OLIGONUCLEOTIDE

(ix) CARACTERISTIQUE ADDITIONELLE:
(A) NOM/CLE: misc_feature
(B) EMPLACEMENT: 1..21
(D) AUTRES RENSEIGNEMENTS: /note= "Stop linker"

(xi) DESCRIPTION DE LA SEQUENCE: SEQ ID NO: 4:

GCGGATCCTA GACTAGTCTA G                                                21

(2) INFORMATION POUR LA SEQ ID NO: 5:

(i) CARACTERISTIQUES DE LA SEQUENCE:
(A) LONGUEUR: 12 paires de bases
(B) TYPE: acide nucléique
(C) NOMBRE DE BRINS: simple
(D) CONFIGURATION: linéaire

(ii) TYPE DE MOLECULE: OLIGONUCLEOTIDE

(ix) CARACTERISTIQUE ADDITIONELLE:
(A) NOM/CLE: misc_feature
(B) EMPLACEMENT: 1..12
(D) AUTRES RENSEIGNEMENTS: /note= "Oligonucleotide contenant
un site BamHI"

(xi) DESCRIPTION DE LA SEQUENCE: SEQ ID NO: 5:

CGCGGATCCG CG 12

(2) INFORMATION POUR LA SEQ ID NO: 6:

(i) CARACTERISTIQUES DE LA SEQUENCE:
(A) LONGUEUR: 14 paires de bases
(B) TYPE: acide nucléique
(C) NOMBRE DE BRINS: simple
(D) CONFIGURATION: linéaire

(ii) TYPE DE MOLECULE: OLIGONUCLEOTIDE

(ix) CARACTERISTIQUE ADDITIONELLE:
(A) NOM/CLE: misc_feature
(B) EMPLACEMENT: 1..14
(D) AUTRES RENSEIGNEMENTS: /note= "Linker SpeI"

(xi) DESCRIPTION DE LA SEQUENCE: SEQ ID NO: 6:

CTAGACTAGT CTAG 14

## Description

La présente invention concerne de nouveaux récepteurs Fc-gamma III humains solubles, leur procédé de préparation, les compositions pharmaceutiques les contenant, leur application comme médicament et leur application diagnostique. Trois classes de récepteur Fc-gamma (RFcγ) qui ont une affinité pour la partie Fc des immunoglobulines G (IgG) humaines ont été décrites. Ils diffèrent par leur affinité pour le ligand, leur distribution cellulaire et leur fonction (J. V. Ravetch et al., Annu. Rev. Immunol., 9, 457, 1991). Les récepteurs Fc-gamma de classe I (RFcγI), porteurs de l'antigène CD64, sont des récepteurs de forte affinité pour les IgG monomériques. Les récepteurs Fc-gamma de classe II (RFcγII), porteurs de l'antigène CD32 et les récepteurs Fc-gamma de classe III (RFcγIII, porteurs de l'antigène CD 16) sont des récepteurs de faible affinité pour les IgG dans les complexes immuns.

Deux types de récepteurs Fc-gamma III, respectivement RFcγIII-1 et RFcγIII-2 qui possèdent des structures et des expressions cellulaires différentes, ont été décrits (J. V. Ravetch et al., J. Exp. Med., 170, 481, 1989). RFcγIII-1 est un récepteur à une seule chaine lié à la surface de la membrane cellulaire par un résidu glycosyl-phosphatidylinositol (GPI) et exprimé exclusivement par les neutrophiles qui représentent le type cellulaire le plus abondant du système immunitaire. RFcγIII-1 fixe les IgG1 et les IgG3 complexées, mais pas les IgG2 et les IgG4 (T. W. J. Huizinga et al., J. Immunol., 142, 2359, 1989). Deux allèles du gène codant pour le RFcγIII-1, appelés respectivement NA1 et NA2, ont été clonés (P. A. Ory et al., J. Clin. Invest., 84, 1688, 1989). RFcγIII-2 est un récepteur membranaire exprimé sur les cellules dites natural killer (NK), les macrophages et les monocytes en culture (J. C. Edberg et al., J. Immunol., 144, 4729, 1990).

Les récepteurs Fc-gamma jouent un rôle important dans la régulation de réactions du système immunitaire (W. H. Fridman et al., Immunol. Rev., 125,49, 1992). Une des remarquables propriétés des récepteurs Fc-gamma repose sur l'existence de formes solubles qui lient les IgG complexées.

Il a été montré que les récepteurs Fc-gamma peuvent être libérés in vitro dans des surnageants de cultures de cellules T et conserver leur affinité spécifique pour le fragment Fc des IgG. Ces récepteurs solubles ont été appelés IgGBF (Immunoglobulin G Binding Factor)(W. H. Fridman et al., Cell. Immunol., 11, 442, 1974). Ces formes solubles circulent également dans les fluides biologiques humains. Ainsi des formes solubles de RFcγIII, que l'on nomme aussi CD16 soluble (CD16s), sont détectées dans des surnageants de cultures de cellules du sang périphérique (W. H. Fridman et al., Immunol. Rev., 56, 51, 1981) aussi bien que dans du sérum humain (T. W. J. Huizinga et al., J. Clin. Invest., 86, 416, 1990) ou dans la salive (C. Teillaud et al., Journal de Parodontologie, Vol 12, N°1, 1, 1992). Ces formes solubles de récepteur RFCγIII sont produites essentiellement par les neutrophiles. Les neutrophiles activés libèrent le CD16 soluble par protéolyse du récepteur lié par un résidu GPI (T. W. J. Huizinga et al., Nature, 333, 667, 1988).

On a montré chez des patients présentant une déficience en gène codant pour RFcγIII-1 que le taux de CD16 soluble est faible ou même non détectable (T. W. J. Huizinga et al., Blood, 10, 1927, 1990). On a aussi observé que des patients atteints de myélome multiple ont une diminution du taux de CD16 soluble dépendante du stade de la maladie (C. Mathiot et al., J. Clin. Inv., 13, N°1, 41, 1993).

Des clones d'ADNc codant pour les récepteurs RFcγIII humains ont été isolés par G. A. Peltz et al., Proc. Natl. Acad. Sci. USA, 86, 1013, 1989 et D. Simmons et al., Nature, 333, 568, 1988. La préparation d'une forme soluble de récepteur RFcγIII constituée du seul domaine extracellulaire qui comprend une chaine polypeptidique de 188 acides aminés a été décrite dans la demande de brevet européenne EP-A-0343950 ainsi que son utilisation dans le traitement du purpura thrombocytopénique immun.

La présente invention concerne de nouveaux récepteurs RFcγIII humains solubles (noté plus loin récepteur RFcγIIIs) dont les propriétés biologiques immunosuppressives inattendues permettent leur utilisation comme médicament, notamment dans le domaine des maladies autoimmunes ou des syndromes autoimmuns, des rejets de greffe et des lymphoproliférations malignes.

La présente invention a donc pour objet un récepteur RFcγIII humain soluble ayant la séquence en acides aminés SEQ ID N°1 : 
Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60
Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val
éventuellement précédée d'une méthionine à l'extrémité N-terminale ainsi que les analogues de cette séquence.

Par analogues, on inclut les séquences modifiées dans la partie C-terminale en position 189 à 194 de la séquence SEQ ID N°1 par substitution, délétion ou addition de un ou plusieurs acides aminés, à l'exception de la séquence dans laquelle les six derniers acides aminés sont éliminés, pour autant que ces produits conservent les propriétés de fixation des IgG1 et des IgG3 caractéristiques du récepteur RFcγIIIs de l'invention.

Ces propriétés de récepteur RFcγIII soluble sont mesurées par des méthodes connues telle que la fixation sur des colonnes d'affinité comprenant une IgG humaine d'isotype particulier dont une illustration est donnée plus loin dans la partie expérimentale ou telle que l'inhibition de la formation de rosettes entre des érythrocytes (RBC) recouvertes d'IgG et des cellules exprimant des récepteurs RFcγIII selon la méthode décrite par exemple par W. H. Fridman et al., Meth. Enzymol, Vol 116, 403, 1986. Le récepteur RFcγIIIs de l'invention peut être préparé par la technologie classique des protéines selon les méthodes connues de la synthèse peptidique ou les méthodes connues du génie génétique qui comprend l'insertion d'une séquence d'ADN codant pour le récepteur RFcIIIs de l'invention dans un vecteur d'expression, la transformation de cellules avec ce vecteur et l'isolement du produit exprimé.

Un des aspect de l'invention concerne un récepteur RFcγIII humain soluble tel qu'obtenu par l'expression dans une cellule hôte d'un ADN codant la séquence en acides aminés SEQ ID N°1 : 
Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60
Asp Ser Gly Glu Tyr Arg Cys Gln Thr Asn Leu Ser Thr Leu Ser Asp
65                  70                  75                  80
 
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val
éventuellement précédée d'une méthionine à l'extrémité N-terminale ainsi que les analogues de cette séquence.

Lorsque le récepteur RFcγIIIs est obtenu par expression dans une cellule hôte, celle-ci est réalisée selon les méthodes connues de génie génétique et de culture cellulaire.

L'expression peut être réalisée dans une cellule hôte procaryote, par exemple E. coli ou dans une cellule eucaryote contenant la séquence codant pour le récepteur RFcγIIIs de l'invention précédée d'une séquence promoteur convenable.

L'invention concerne plus précisément un récepteur RFcγIII humain soluble tel qu'obtenu par l'expression dans une cellule hôte eucaryote d'un ADN codant la séquence en acides aminés SEQ ID N°1 :

Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60

Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val

Les cellules hôte eucaryotes comprennent les levures ainsi que les cellules d'organismes supérieurs, par exemple des cellules de mammifères. Les cellules de mammifères sont par exemple des fibroblastes tels que les cellules L de souris, des cellules de hamster telles que les cellules CHO ou les cellules BHK ou des cellules COS de singe.

L'invention concerne aussi une séquence d'ADN comprenant une séquence d'ADN codant pour le récepteur RFcγIII humain soluble ayant la séquence en acides aminés SEQ ID N°1 ou une séquence analogue de celle-ci. L'invention concerne notamment une séquence d'ADN codant pour le récepteur RFcγIII humain soluble ayant la séquence en acides aminés SEQ ID N°1 ou une séquence analogue de celle-ci.

L'invention concerne spécialement une séquence d'ADN codant pour le récepteur RFcγIIIs de l'invention constituée essentiellement de la séquence nucléotidique SEQ ID N°2. La séquence SEQ ID N°2 est obtenue à partir d'une séquence d'ADNc dont le clonage a été décrit (D. Simmons et al., déjà cité) et dans laquelle une séquence contenant un codon de terminaison de la traduction est introduite par les méthodes de mutagénèse dirigée. La séquence SEQ ID N°2 qui comprend 672 nucléotides contient une région codante pour le récepteur RFcγIIIs. La séquence déduite en acides aminés est donnée en dessous (SEQ ID N°3). La séquence en acides aminés du récepteur RFcγIIIs, commencant à la position +1, comprend 194 acides aminés et est précédée d'une séquence signal peptide ayant 18 acides aminés.

L'invention a également pour objet des vecteurs d'expression comprenant une séquence d'ADN codant pour un récepteur RFcγIII humain soluble selon l'invention ainsi que des hôtes transformés avec un vecteur ci-dessus.

L'invention concerne particulièrement un hôte qui est une cellule eucaryote.

Un autre aspect de l'invention concerne un procédé qui comprend l'expression du récepteur RFcγIII humain soluble dans une cellule hôte transformée ci-dessus et notamment un procédé dans lequel la cellule hôte est une cellule eucaryote et dont un exemple est décrit plus loin.

La présente invention a également pour objet des anticorps monoclonaux dirigés contre le récepteur RFcγIII humain soluble selon l'invention ou contre un fragment immunogène de celui-ci. Les anticorps sont préparés selon les méthodes connues et peuvent être utilisés par exemple pour diagnostiquer des pathologies caractérisées par une modification des taux de récepteurs FcγIII solubles ou pour le dosage du récepteur RFcγIIIs de l'invention dans des fluides biologiques en utilisant le récepteur RFcγIIIs ci-dessus comme molécule standard, par exemple dans un test ELISA. L'invention a ainsi également pour objet une composition de diagnostic comprenant un ou plusieurs anticorps selon l'invention.

Le récepteur RFcγIIIs de l'invention a de remarquables propriétés biologiques immunosuppressives, en particulier une activité antiproliférative, une activité inhibitrice de la production des immunoglobulines ainsi qu'une activité anticytotoxicité cellulaire de type Natural Killer (NK) comme le montrent les résultats donnés plus loin.

Ces propriétés rendent le récepteur RFcIIIs de l'invention utilisable dans le traitement de maladies autoimmunes telles que par exemple le diabète auto-immun, la polyarthrite rhumatoïde, le lupus érythrémateux dissiminé ou la sclérose en plaques ainsi que dans le traitement de syndromes autoimmuns consécutifs à des traitements immunostimulants, par exemple à un traitement par de l'interleukine 2. Le produit de l'invention peut être également utile dans le traitement de lymphoproliférations malignes en particulier les lymphomes et les myèlomes B ou dans la prévention de rejets de greffe consécutives à des transplantations d'organes et dans le traitement de lymphoproliférations malignes.

La présente invention a donc pour objet à titre de médicament, le récepteur RFcγIII humain soluble de l'invention.

L'invention s'étend aux compositions pharmaceutiques renfermant comme principe actif un médicament défini ci-dessus et concerne particulièrement les compositions pharmaceutiques pour moduler la prolifération cellulaire, pour moduler la cytotoxicité de type NK ou pour moduler la production d'anticorps.

Le principe actif peut être incorporé à des excipients usuels pour la préparation des compositions pharmaceutiques ci-dessus. Les compositions de l'invention peuvent être administrées par voie parentérale, orale ou locale.

Les figures ci-annexées illustrent certains aspects de l'invention :

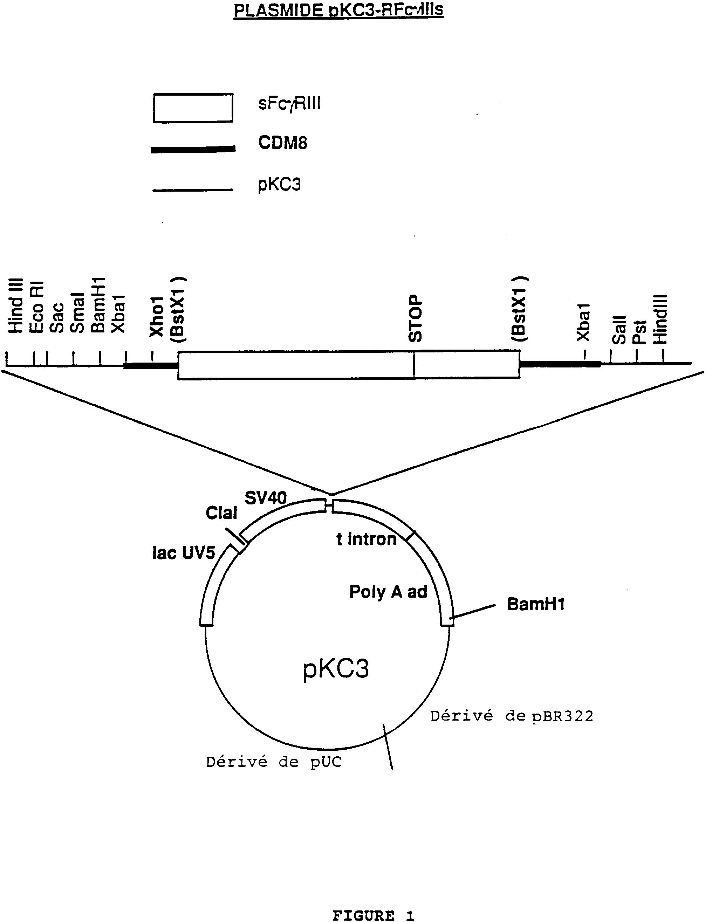

La figure 1 représente le schéma du plasmide pKC3-RFcγIIIs contenant l'insert de la séquence SEQ ID N°2.

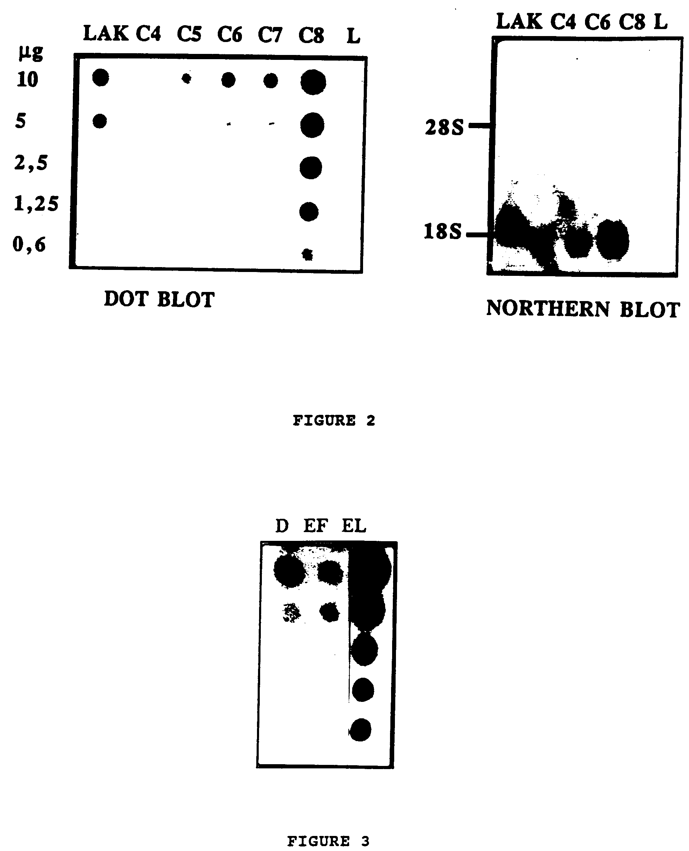

La figure 2 représente la détection de l'ARN spécifique du RFcγIIIs dans les clones résistants sélectionnés C4 à C8 avec des cellules LAK comme témoin positif et des cellules L comme témoin négatif, par la méthode Dot blot ou la méthode Northern blot à l'aide d'une sonde radioactive spécifique.

La figure 3 représente le titrage du RFcγIIIs dans l'effluent (EF) et dans l'éluat acide (EL) de la colonne d'affinité 3G8 par rapport au bouillon récolté (D), par immunoempreinte à l'aide d'un anticorps monoclonal BW209/2 radiomarqué.

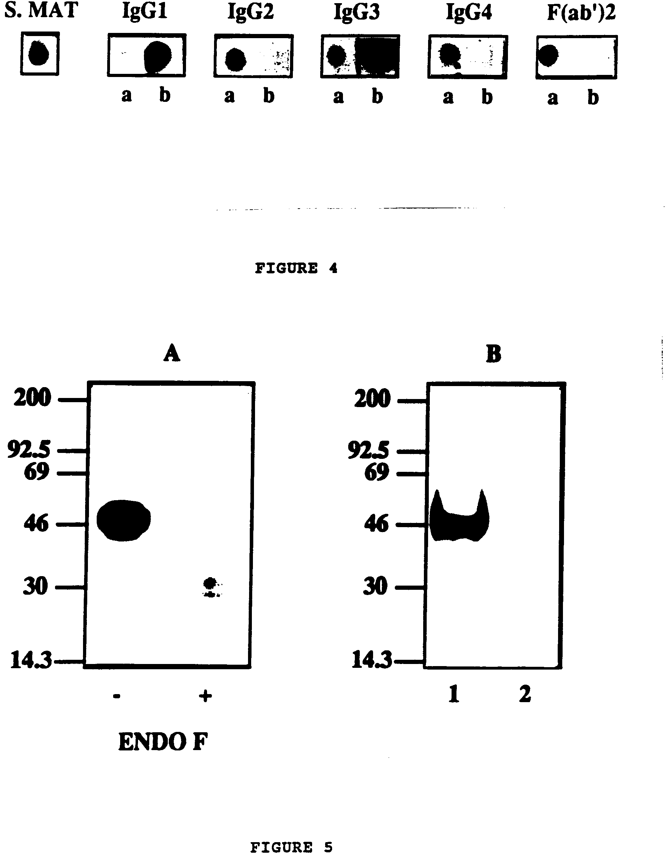

La figure 4 représente le titrage de la spécificité du RFcγIIIs purifié à fixer les IgG (IgG1 à IgG4) ainsi que des fragments F(ab')₂ d'IgG, par immunoempreinte à l'aide de l'anticorps BW209/2 radiomarqué dans l'effluent (a) et dans l'éluat (b) respectifs d'une colonne d'IgG donnée ou de fragment F(ab')₂.

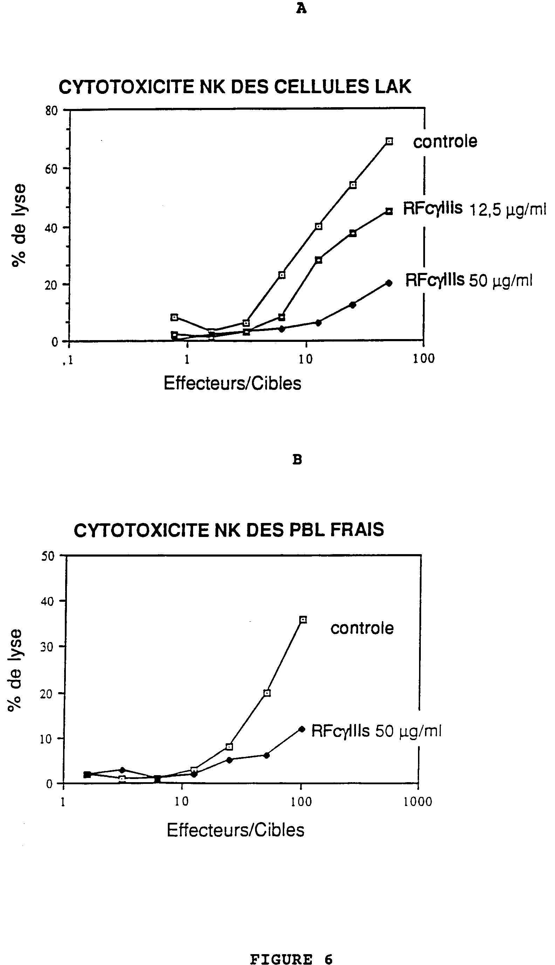

La figure 5 représente la SDS-PAGE du RFcγIIIs purifié. Le RFcγIIIs radiomarqué est détecté par autoradiographie avant ou après déglycosylation par Endo F (figure 5A) ou par Western blot (figure 5B, ligne 1) à l'aide de l'anticorps BW209/2 radiomarqué comparativement à un contrôle-témoin (figure 5B, ligne 2).

La figure 6 représente l'effet inhibiteur du RFcγIIIs sur la cytotoxicité NK des cellules effecteurs LAK (figure 6A) ou des cellules effecteurs PBL (figure 6B) exprimée en % de lyse de cellules cibles K562 mesurée par le relarguage de Cr51 en fonction de la concentration relative des cellules effecteurs cellules effecteurs (LAK ou PBL)/cibles et de la concentration en RFcγIIIs.

Les exemples suivants illustrent l'invention sans la limiter.

### EXEMPLE 1 : production d'un récepteur FcγIII humain soluble (RFcγIIIs) dans des cellules d'eucaryotes.

Des cellules d'eucaryotes qui sécrètent en permanence un récepteur RFcγIIIs sont obtenues par co-transfection avec un vecteur portant un marqueur de sélection et un vecteur d'expression portant une séquence d'ADN codant pour le récepteur RFcγIIIs et un promoteur compatible avec la souche hôte. Les activités biologiques sont déterminées sur le produit isolé et purifié à l'homogénéité.

### A - Construction de la séquence codante :

La séquence d'ADN codant pour le récepteur RFcγIIIs est obtenue à partir de l'ADNc codant pour un récepteur RFcγIII exprimé dans les PMN dans lequel on introduit une séquence nucléotidique notée "stop linker" contenant un codon TAG de terminaison de la traduction :
Le plasmide pCD16 décrit par D. Simmons (déjà cité) comprend un insert d'ADNc de 893 bp dans lequel on introduit le "stop linker" GCGGATCCTAGACTAGTCTAG (SEQ ID N°4) à la position 652, selon les méthodes connues de mutagénèse dirigée. pCD16 est successivement digéré par l'endonucléase de restriction KpnI, traité avec de la T4-DNA polymérase pour enlever l'extrémité 3' protubérante, puis ligaturé à un oligonucléotide ayant la séquence CGCGGATCCGCG (SEQ ID N°5) contenant un site BamHI. Le fragment obtenu est ensuite cloné dans un plasmide portant un site BamHI unique. Pour créer le codon stop TAG après le site BamHI, un site SpeI est d'abord éliminé. Le site BamHI est clivé puis les extrémités créées sont remplies à l'aide du fragment Klenow de la DNA polymérase de E. coli. Une séquence "linker SpeI" CTAGACTAGTCTAG (SEQ ID N°6), contenant un site de restriction de l'enzyme SpeI, est insérée. On obtient l'insert de 672 bp ayant la séquence nucléotidique SEQ ID N°2.

L'insert a été sous cloné aux sites BstX1 du plasmide CDM8 (B. Seed, Nature, 329, 840, 1987). Après digestion avec l'enzyme de restriction Xba1, le fragment isolé est inséré dans le site polylinker du plasmide pKC3 dérivé du plasmide pKO-néo (K. Van Doren et al., J. Virol., 50, 606, 1984) et dont la structure est montrée à la figure 1.
Le plasmide obtenu est dénommé pKC3-RFcγIIIs (figure 1).

### B - Transfection et sélection d'une cellule transformée.

Le plasmide pKC3-RFcγIIIs est introduit par cotransfection avec le plasmide pSV2-néo (K. Van Doren et al., déjà cité) dans des fibroblastes murins (cellules L) par la méthode de coprécipitation au phosphate de calcium :
Des boites de Pétri de 60 mm de diamètre sont ensemencées avec 106 cellules L cultivées en milieu DMEM (Gibco BRL) complété avec 10 % de sérum de veau fétal (SVF) (Flow laboratories), 100 UI/ml de pénicilline (Gibco BRL), 100 µg/ml de streptomycine (Gibco BRL), 2mM de L-glutamine (Gibco BRL). Après une nuit d'incubation à 37°C en étuve à 7 % de CO2, les cellules sont cotransfectées avec 10 µg de plasmide pKC3-RFcγIIIs et 100 ng de plasmide pSV2-neo. Après trois jours de culture, les cellules sont placées en milieu sélectif contenant 1 mg/ml d'antibiotique G-418 (Geneticin, G-418 sulfate, Gibco BRL) puis sont maintenues dans un milieu contenant 500 µg/ml de G-418.

L'ARN total de clones résistants au milieu de sélection est isolé et analysé selon les techniques de Dot blot et de Northern blot. L'ARN spécifique du récepteur RFcγIIIs est détecté à l'aide d'une sonde spécifique constituée du fragment Xba1-Xba1 de pKC3-RFcγIIIs marquée au ³²P avec [α-³²P) dCTP (370 MBq/ml) par la méthode "random primer" décrite par A. P. Feinberg et al. Anal. Biochem 132, 6, 1983.

On utilise comme contrôle positif l'ARN de lymphocytes humains cultivés en présence d'interleukine 2 (cellules LAK) et comme contrôle négatif des cellules L non transfectées.

La figure 2 montre les résultats obtenus avec cinq clones résistants désignés C4 à C8. Le clone C8, noté plus loin IIIC8, qui renferme le plus grand nombre de copies d'ARN codant pour le récepteur RFcγIIIs est sélectionné pour les travaux ultérieurs.

### C - Production du récepteur RFcγIIIs.

Le clone IIIC8 est cultivé dans un bioréacteur de type Acusyst Junior (Endotronics) dans le milieu DMEM complété à 5 % de SVF. Le bioréacteur de 1,1 m² est ensemencé avec 3X10⁸ cellules et un flux continu de milieu DMEM à 5 % de SVF est installé au débit de 1 ml/h. La récolte du bouillon est effectuée du jour 12 au jour 22.

### EXEMPLE 2 : purification du récepteur RFcγIIIs.

60 ml du bouillon récolté ci-dessus sont dialysés pendant 18 h à 4°C contre du tampon Tris-Base 20 mM à pH 7,6, puis passés sur une colonne de 0,5 ml de BSA-Sépharose 4B (Pharmacia) et chromatographiés au débit de 15 à 20 ml/h, à 4°C sur une colonne contenant 0,5 ml de Sepharose 4B (Pharmacia) couplé à l'anticorps monoclonal 3G8 qui est un anticorps de souris anti-CD16 d'isotype IgG1, k décrit par H. B. Fleit et al. (Proc. Natl. Acad. Sci 79, 3275, 1982) et équilibré dans le tampon Tris-Base 20 mM à pH 7,6. L'effluent est passé à nouveau deux fois sur la même colonne. La colonne est lavée avec 40 ml du même tampon puis éluée au débit de 3 ml/h avec un tampon glycine-HCl 0,2 M à pH 2,8. On obtient 2 ml d'éluat acide que l'on neutralise avec un tampon Tris-Base 2M à pH 9 que l'on soumet aux analyses suivantes :

### a) Teneur en protéine

L'éluat acide obtenu renferme 72 ± 45 µg de protéine titrée selon le micro essai de Bradford (Bio-Rad Protein Assay).

### b) Teneur en récepteur RFcγIIIs

La mise en évidence du récepteur RFcγIIIs est faite par empreinte directe sur une membrane de nitrocellulose en utilisant l'anticorps monoclonal BW209/2 qui est un anticorps monoclonal de souris anti-CD16, d'isotype IgG2a décrit par D. Simmons et al. (déjà cité) et que l'on a radiomarqué à l'iode 125.

Les résultats de l'immunoempreinte effectuée simultanément sur le bouillon de départ (D), sur l'effluent (EF) et sur l'éluat acide (EL) de la colonne d'affinité permettent d'évaluer le rendement de la chromatographie d'affinité entre 16 et 30 % de la teneur initiale en récepteur RFcγIIIs présent dans le bouillon récolté (figure 3).

### EXEMPLE 3 : caractérisation du récepteur RFcγIIIs

### A - Spécificité isotypique de la fixation des IgG

La spécificité de fixation des IgG du récepteur RFcγIIIs de l'invention est déterminée par chromatographie d'affinité sur une colonne d'IgG humaine d'isotype déterminé.

Le bouillon récolté, dialysé et passé sur la colonne de BSA-Sépharose 4B obtenu à l'exemple 2 est chromatographié sur une colonne contenant 0,5 ml de Sépharose 4B auquel est couplé 3 mg d'IgG humaine monoclonale d'isotype respectif IgG1, IgG2-kappa, IgG3-kappa, IgG4-kappa ou de fragments F(ab')₂ d'IgG polyclonale préparés par digestion d'une IgG humaine par la pepsine suivie d'une purification sur une colonne de protéine A-sépharose CL-4B (Pharmacia). Chaque colonne d'immunoadsorbant est lavée avec un tampon Tris-Base 20 mM à pH 7,6 puis éluée avec un tampon glycine-HCl 0,2M à pH 2,8. Chaque éluat acide obtenu respectivement est neutralisé avec un tampon Tris-Base 2M à pH 9.

50 µl du matériel de départ, de chaque effluent et de chaque éluat acide correspondant après neutralisation sont respectivement testés en empreinte directe avec l'anticorps monoclonal anti-CD16 BW209/2 radiomarqué, comme indiqué à l'exemple 2.

Les résultats donnés à la figure 4 montrent des spots intenses dans les éluats (b) correspondant aux immuno-adsorbants d'IgG1 et d'IgG3 immobilisées tandis que les éluats (b) des immunoadsorbants d'IgG2, IgG4 ou de F(ab')₂ sont négatifs et que les effluents (a) donnent des spots faibles inférieurs à celui du matériel de départ (S. MAT). Ces résultats indiquent que le récepteur RFcγIIIs de l'invention se fixe aux IgG1 et IgG3 insolubilisées et ne se fixe pas de façon significative aux IgG2 et IgG4.

### B - Homogénéité

Le récepteur RFcγIIIs purifié ci-dessus est analysé par électrophorèse SDS-PAGE sur des gels à 10 % de polyacrylamide selon la méthode de Laemmli (Nature, 227, 680, 1970) dans un système de gel minilab (Mini-Protean II Electrophoresis cell, Bio-Rad) suivie d'une révélation par autoradiographie ou par immuno détection :

### a) SDS-PAGE et autoradiographie :

On utilise des échantillons d'éluat acide positif à l'immunoempreinte obtenu à l'exemple 2 que l'on dialyse contre du tampon PBS puis radiomarque à l'iode 125 à l'aide d'iodure de sodium radioactif par la méthode à la chloramine T. L'éluat radiomarqué est éventuellement ensuite déglycosylé : 0,1 ml d'éluat radiomarqué est dilué avec 9 volumes de tampon phosphate de sodium 100 mM à pH 6,1 contenant 50 mM d'EDTA, 1 % de Triton X-100 et 1 % de 2-mercaptoéthanol, maintenu à 100°C pendant 5 minutes, puis traité par une unité de ENDO F (Endo-β-N-acétyl glucosoaminidase F, Boehringer Mannheim Biochemica) à 37°C pendant 18 heures puis par l'acide trichloracétique à 10 %. Le précipité obtenu est lavé avec de l'acétone à -20°C, porté à 100°C dans du tampon Tris-HCl 80 mM à pH 6,8 contenant 100 mM de DTT, 2 % de SDS, 10 % de glycérol et 0,01 % de bleu de bromophénol.

L'échantillon radiomarqué avant déglycosylation (- EndoF) révèle par autoradiographie une seule bande correspondant à un poids moléculaire apparent de 48kDa alors que l'échantillon radiomarqué puis déglycosylé (+ Endo F) révèle deux bandes à 26 et 30 kDa (figure 5A).

### b) SDS-PAGE et Western Blotting :

30 µl d'éluat acide obtenu à l'exemple 2 sont portés à ébullition pendant trois minutes dans le tampon SDS d'échantillon puis soumis à la SDS-PAGE pendant 40 minutes à 200 volts à la température ambiante. Le gel est lavé avec un tampon Tris-base 25 mM à pH 8,3 contenant de la glycine 192 mM et 2 % de méthanol, puis transféré sur une membrane de nitrocellulose (BA-85, 0, 45 µm, Schleicher & Schüll) pendant une heure à 100 volts en utilisant un système de transfert (Bio-Rad, Mini Trans-Blot Electrophoretic Transfer cell). La membrane de nitrocellulose est ensuite saturée pendant 45 minutes à 40°C dans un tampon Tris-HCl 10 mM à pH 7,4 contenant NaCl 150 mM et 5 % de BSA (Western buffer, noté WB) et incubée pendant 18 heures à 4°C avec l'anticorps monoclonal anti-CD16 BW209/2 marqué à l'iode 125, à la concentration de 10⁵ cpm par ml pour 10 cm² de nitrocellulose. La membrane est ensuite lavée successivement deux fois dans le tampon WB, une fois dans le tampon WB contenant 0,05 % de Nonidet P40 (Fluka) et encore deux fois dans le tampon WB, puis est exposée pendant une nuit à -70°C à un film Kodak X-OMAT S (Eastman Kodak) pour autoradiographie.

On traite de la même façon un éluat acide obtenu à partir de cultures de cellules L non transfectées, à titre de contrôle témoin.

Les résultats donnés à la figure 5B montrent que le RFcγIIIs purifié (ligne 1) migre comme une glycoprotéine ayant un PM apparent de 48 kDa tandis qu'aucune détection n'est observée avec le contrôle témoin (ligne 2).

### EXEMPLE 4 : activité immunosuppressive du récepteur RFcγIIIs

Le récepteur RFcγIIIs purifié ci-dessus présente une activité immunosuppressive qui est évaluée sur des cellules activées in vitro par mesure de sa capacité à inhiber respectivement :
- la prolifération de lymphocytes stimulés;
- la production d'anticorps par les lymphocytes;
- la cytotoxicité des cellules NK (natural killer).

### A - Inhibition de la prolifération des PBMC stimulés par le mitogène pokeweed (PWM)

Les cellules mononucléaires du sang périphériques (PBMC) de donneur sains sont préparés par séparation à l'aide d'un gradient de densité de Ficoll-Isopaque. Les PBMC décongélés sont cultivés dans des plaques de 96 puits (Falcon 3072) à 37°C dans une atmosphère humide à 7 % de CO2. Les PBMC sont ajustés à 2 X 10⁶ cellules /ml dans du milieu RPMI-1640 (Gibco) complété avec 10 % de sérum de veau foetal (SVF) (Flow Laboratories), 100 UI/ml de pénicilline, 100 µg mM/ml de streptomycine, 1 % de L-glutamine, 1 % d'acides aminés non essentiels (Gibco) et de 0,05 M/l de 2-mercaptoéthanol (Gibco).

2 X 10⁵ cellules sont réparties dans les plaques de 96 puits et incubées pendant 6 jours dans un volume final de 200 µl de milieu de culture contenant du PWM (IBF) à la concentration de 0,1 µg/ml (contrôle stimulé) ou ne contenant pas de PWM (contrôle non stimulé) et en présence de différentes doses de RFcγIIIs purifié à l'exemple 2 ou de tampon PBS (contrôle). Chaque culture est faite en trois répliques. La prolifération des PBMC est mesurée au sixième jour de culture par incorporation de 1 µCi de thymidine tritiée (Amersham). Après 6 heures d'incubation à 37°C en présence de 7 % de CO2, la quantité de radioactivité incorporée est mesurée avec un compteur béta (Pharmacia).

Le pourcentage d'inhibition de la prolifération est calculé par rapport au contrôle stimulé.

Les résultats obtenus dans deux expériences séparées sont donnés dans le tableau suivant :

**Tableau 1**

| PWM dose de RFcγIIIs µg / ml | | Thymidine incorporée cpm | % inhibition |
|---|---|---|---|
| 1° expérience | | | |
| - | - | 153 | / |
| + | - | 38393 | / |
| + | 0,012 | 37600 | 0 |
| + | 1,5 | 22558 | 41 |
| + | 6,2 | 4376 | 89 |

| 2° expérience | | | |
|---|---|---|---|
| - | - | 542 | / |
| + | - | 40798 | / |
| + | 0,012 | 43410 | 0 |
| + | 1,5 | 32207 | 21 |
| + | 6,2 | 62 | 100 |

A la dose de 6, 2 µg/ml en RFcγIIIs, l'activation de la prolifération des PBMC stimulés par PWM est totalement inhibée.

### B - Inhibition de la production d'anticorps par les PBMC stimulés par le PWM

Les cultures cellulaires sont réalisées comme précédemment en trois répliques.

La production d'IgG et d'IgM est mesurée au sixième jour dans les surnageants à l'aide d'un test ELISA spécifique des IgG ou des IgM, selon la méthode décrite par E. Thibaut et al. J. Immunol. Meth., 104, 15, 1987.

Le pourcentage d'inhibition de la production d'anticorps est calculé par rapport au contrôle stimulé.

Les résultats obtenus dans deux expériences sont donnés dans le tableau suivant :

**Tableau 2**

| PWM dose de RFcγIIIs µg / ml | | IgM ng/ml | % inhibition | IgG ng/ml | % inhibition |
|---|---|---|---|---|---|
| 1° experience | | | | | |
| - | - | 100 | / | 18 | / |
| + | - | 582 | / | 192 | / |
| + | 0,012 | 492 | 15 | 100 | 59 |
| + | 1,5 | 64 | 89 | 10 | 95 |
| + | 6,2 | 0 | 100 | 12 | 95 |

| 2° expérience | | | | | |
|---|---|---|---|---|---|
| - | - | 170 | / | 20 | / |
| + | - | 287 | / | 156 | / |
| + | 0,012 | 190 | 34 | 78 | 50 |
| + | 1,5 | 0 | 100 | 0 | 100 |
| + | 6,2 | 0 | 100 | 0 | 100 |

A la dose de 1,5 µg/ml en RFCyIIIs, la production d'IgG ou d'IgM par les PBMC stimulés par PWM est totalement inhibée.

### C - Inhibition de la cytotoxicité de type NK

L'étude est réalisée sur des cellules mononuclées circulantes humaines incubées ou non en présence d'IL2 et dont on détermine l'effet cytotoxique vis à vis de cellules cibles tumorales K562 qui est une lignée érythroleucémique sensible aux cellules NK, par mesure du relarguage de Cr51 en 4 heures.

Les cellules mononucléaires du sang périphérique (PBL) de donneurs sains sont préparés à l'aide d'un gradient de Ficoll-Isopaque, lavés et ajustés à 10 X 10⁶ cellules /ml dans du milieu RPMI 1640 (Gibco) complété avec 10 % de SVF, 1 % de L-glutamine, 100 UI/ml de pénicilline et 100 µg/ml de streptomycine (noté RPMI 10 % SVF).

Les cellules LAK sont obtenues par culture des PBL pendant trois jours à 37°C en atmosphère humide à 7 % de CO2 en présence de 1000 u/ml d'IL2.

Les cellules effecteurs PBL ou LAK sont incubées à la concentration de 10 X 10⁶ cellules/ml dans du milieu RPMI 10 % SVF pendant 2 heures à 37°C en présence de différentes doses de récepteur RFcγIIIs purifié à l'exemple 2. Les cellules sont ensuite lavées et remises en suspension à 5 X 10⁶ cellules/ml dans le même milieu.

100 µl de cellules effecteurs PBL ou LAK contenant 5 X 10⁶ à 7 X 10⁴ cellules sont incubés avec 100 µl de cellules cibles (5 X 10⁴ cellules) dans des plaques en V à 96 puits pendant 4 heures à 37°C. Après centrifugation à 2000 rpm pendant 5mn, 100 µl de surnageant sont prélevés dans lequel on mesure la radioactivité présente.

Les résultats sont exprimés en pourcentage de lyse des cellules cibles. Les figures 6A et 6B montrent l'inhibition de la lyse respectivement par les cellules LAK et par les PBL en présence de doses variables de récepteur RFcγIIIs purifié en fonction de la concentration relative effecteurs/cibles.

## Revendications

**1)** Récepteur RFcγIII humain soluble ayant la séquence en acides aminés SEQ ID N°1 : 
Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60
Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val
 éventuellement précédée d'une méthionine à l'extrémité N-terminale ainsi que les analogues de cette séquence.

**2)** Récepteur RFcγIII humain soluble tel qu'obtenu par l'expression dans une cellule hôte d'un ADN codant la séquence en acides aminés SEQ ID N°1 : 
Arg Thr Glu Asp Leu Pro Lys Ala Val Val Phe Leu Glu Pro Gln Trp
1               5                   10                  15
 
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60
Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val
 éventuellement précédée d'une méthionine à l'extrémité N-terminale ainsi que les analogues de cette séquence.

**3)** Récepteur RFcγIII humain soluble tel qu'obtenu par l'expression dans une cellule hôte eucaryote d'un ADN codant la séquence en acides aminés SEQ ID N° 1 :

Arg Thr Glu Asp Leu Pro Lys Ala Val Val Phe Leu Glu Pro Gln Trp
1               5                   10                  15
Tyr Ser Val Leu Glu Lys Asp Ser Val Thr Leu Lys Cys Gln Gly Ala
            20                  25                  30
Tyr Ser Pro Glu Asp Asn Ser Thr Gln Trp Phe His Asn Glu Ser Leu
        35                  40                  45
Ile Ser Ser Gln Ala Ser Ser Tyr Phe Ile Asp Ala Ala Thr Val Asn
    50                  55                  60

Asp Ser Gly Glu Tyr Arg Cys Gln Thr Asn Leu Ser Thr Leu Ser Asp
65                  70                  75                  80
Pro Val Gln Leu Glu Val His Ile Gly Trp Leu Leu Leu Gln Ala Pro
                85                  90                  95
Arg Trp Val Phe Lys Glu Glu Asp Pro Ile His Leu Arg Cys His Ser
            100                 105                 110
Trp Lys Asn Thr Ala Leu His Lys Val Thr Tyr Leu Gln Asn Gly Lys
        115                 120                 125
Asp Arg Lys Tyr Phe His His Asn Ser Asp Phe His Ile Pro Lys Ala
    130                 135                 140
Thr Leu Lys Asp Ser Gly Ser Tyr Phe Cys Arg Gly Leu Val Gly Ser
145                 150                 155                 160
Lys Asn Val Ser Ser Glu Thr Val Asn Ile Thr Ile Thr Gln Gly Leu
                165                 170                 175
Ala Val Ser Thr Ile Ser Ser Phe Ser Pro Pro Gly Ala Asp Pro Arg
            180                 185                 190
Leu Val

**4)** Séquence d'ADN comprenant une séquence d'ADN codant pour le récepteur RFcγIII humain soluble selon la revendication 1.

**5)** Séquence d'ADN codant pour le récepteur RFcγIII humain soluble selon la revendication 1.

**6)** Séquence d'ADN selon la revendication 5 constituée essentiellement de la séquence nucléotidique SEQ ID N°2.

**7)** Vecteurs d'expression comprenant une séquence d'ADN codant pour un récepteur RFcγIII humain soluble selon la revendication 1.

**8)** Hôtes transformés avec un vecteur selon la revendication 7.

**9)** Hôte selon la revendication 8 qui est une cellule eucaryote.

**10)** Procédé qui comprend l'expression du récepteur RFcγIII humain soluble dans une cellule hôte transformée par un ADN codant pour un récepteur RFcγIII humain soluble selon la revendication 1.

**11)** Procédé selon la revendication 10 dans lequel la cellule hôte est une cellule eucaryote.

**12)** Anticorps monoclonaux dirigés contre le récepteur RFcγIII humain soluble selon la revendication 1 ou contre un fragment immunogène de celui-ci.

**13)** Composition de diagnostic comprenant un ou plusieurs anticorps selon la revendication 12.

**14)** A titre de médicament, le récepteur RFcγIII humain soluble selon la revendication 1.

**15)** Compositions pharmaceutiques renfermant comme principe actif un médicament selon la revendication 14.

**16)** Compositions pharmaceutiques selon la revendication 15 pour moduler la prolifération cellulaire.

**17)** Compositions pharmaceutiques selon la revendication 15 pour moduler la cytotoxicité de type NK.

**18)** Compositions pharmaceutiques selon la revendication 15 pour moduler la production d'anticorps.
